# EUROPEAN PATENT SPECIFICATION

(11) **EP 3 487 847 B1**
(45) Date of publication and mention of the grant of the patent: **02.10.2024**
(21) Application number: 17831584.2
(22) Date of filing: 13.07.2017
(51) Int. Cl.: B01J 27/128, B01J 27/32, B01J 38/12, C07D 307/68, B01D 3/00, B01D 11/04

(54) **A FURAN-2,5-DICARBOXYLIC ACID PURGE PROCESS**
REINIGUNGSVERFAHREN FÜR FURAN-2,5-DICARBONSÄURE
PROCESSUS DE PURGE D'ACIDE FURANE-2,5-DICARBOXYLIQUE

(30) Priority: 22.07.2016 US 201615217319
(43) Date of publication of application: 29.05.2019
(73) Proprietor: Eastman Chemical Company, Kingsport, TN 37660 (US)
(72) Inventor: FONTENOT, Kevin, John, Jonesborough, TN 37659 (US); JANKA, Mesfin, Ejerssa, Kingsport, TN 37660 (US); PARKER, Kenny, Randolph, Afton, TN 37616 (US); SHAIKH, Ashfaq, Shahanawaz, Kingsport, TN 37660 (US); PARTIN, Lee, Reynolds, Lexington, KY 40511 (US); SUMMER, Charles, Edwan, Jr., Kingsport, TN 37664 (US); KIRK, Shane, Kipley, Church Hill, TN 37642 (US); MOODY, Paula, Johnson City, TN 37604 (US); BOWERS, Bradford, Russell, Chuckey, TN 37641 (US); MORROW, Michael, Charles, Kingsport, TN 37664 (US)
(74) Representative: Ricker, Mathias
(86) International application number: PCT/US2017/041842
(87) International publication number: WO 2018/017382

(56) References cited:
- WO-A1-2012/161973
- WO-A2-2007/092183
- WO-A2-2010/132740
- WO-A2-2012/161970
- US-A1- 2002 193 631
- US-A1- 2006 205 977
- US-A1- 2012 302 771

## Description

### BACKGROUND OF THE INVENTION

Aromatic dicarboxylic acids such as terephthalic acid and isophthalic acid or their di-esters, dimethyl terephthalate as for example, are used to produce a variety of polyester products, important examples of which are poly (ethylene terephthalate) and its copolymers. The aromatic dicarboxylic acids are synthesized by the catalytic oxidation of the corresponding dialkyl aromatic compounds which are obtained from fossil fuels (US 2006/0205977 A1). Esterification of these diacids using excess alcohol produces the corresponding di-esters (US2010/0210867A1) .There is a growing interest in the use of renewable resources as feed stocks for the chemical industries mainly due to the progressive reduction of fossil reserves and their related environmental impacts.

Furan-2,5-dicarboxylic acid (FDCA) is a versatile intermediate considered as a promising closest biobased alternative to terephthalic acid and isophthalic acid. Like aromatic diacids, FDCA can be condensed with diols such as ethylene glycol to make polyester resins similar to polyethylene terephthalate (PET) (Gandini, A.; Silvestre, A. J; Neto, C. P.; Sousa, A. F.; Gomes, M., J. Poly. Sci. A 2009, 47, 295). FDCA has been prepared by oxidation of 5-(hydroxymethyl) furfural (5-HMF) under air using homogenous catalysts (US2003/0055271 A1 and Partenheimer, W.; Grushin, V. V., Adv. Synth. Catal. 2001, 343, 102-111.) but only a maximum of 44.8% yield using a Co/Mn/Br catalyst system and a maximum of 60.9 % yield was reported using Co/Mn/Br/Zr catalysts combination. Recently, we reported a process for producing furan-2,5-dicarboxylic acid (FDCA) in high yields by liquid phase oxidation of 5-HMF or its derivatives using a Co/Mn/Br catalyst system that minimizes solvent and starting material loss through carbon burn (U.S. Patent Application Numbers 13/228803, 13/228809, 13/228816, and 13/228799).

WO 2012/161970, WO 2012/161973, and US 2012/302771 disclose oxidation processes to produce a crude carboxylic acid product.

The present invention relates to a process for producing a carboxylic acid composition, wherein the process is defined as set out in the claims.

Disclosed is a method for recovering a portion of oxidation solvent, a portion of oxidation catalyst, and removing a portion of oxidation by-products and raw material impurities from a solvent stream generated in a process to make furan-2,5-dicarboxylic acid (FDCA). The process comprises oxidizing a feed stream comprising at least one oxidizable compound selected from the following group: 5-(hydroxymethyl)furfural (5-HMF), 5-(chloromethyl)furfural (5-CMF), 2,5-dimethylfuran (2,5 -DMF), 5-HMF esters (5-R(CO)OCH₂-furfural where R = alkyl, cycloalkyl and aryl), 5-HMF ethers (5-R'OCH₂-furfural, where R' = alkyl, cycloalkyl and aryl), 5-alkyl furfurals (5-R"-furfural, where R" = alkyl, cycloalkyl and aryl), alkylcarboxylate of methyfuran (5-R‴O(CO)-methylfuran, where R‴ = alkyl, cycloalkyl), alkylcarboxylate of alkoxyfuran (5-RʺʺO(CO)-OR‴ʺfuran, where Rʺʺ = alkyl, cycloalkyl and aryl and R‴ʺ = alkyl, cycloalkyl and aryl), mixed feed-stocks of 5-HMF and 5-HMF esters and mixed feed-stocks of 5-HMF and 5-HMF ethers, mixed feed-stocks of 5-HMF and 5-alkyl furfurals, mixed feed-stocks of 5-HMF and 5-CMF, mixed feed-stocks of 5-HMF and 2,5-DMF, mixed feed-stocks of 5-HMF and alkylcarboxylate of methyfuran, mixed feed-stocks of 5-HMF and alkylcarboxylate of alkoxyfuran to generate a crude carboxylic acid slurry comprising furan-2,5-dicarboxylic acid (FDCA) in an oxidation zone, cooling a crude carboxylic acid slurry in a cooling zone to generate a cooled crude carboxylic acid slurry, removing impurities from a cooled crude carboxylic acid slurry in a solid-liquid separation zone to form a low impurity carboxylic acid stream and a mother liquor stream, routing at least a portion of the mother liquor stream to a mother liquor purge zone to generate a recycle oxidation solvent stream, a recycle catalyst rich stream, a raffinate stream, and an impurity rich waste stream.

### BRIEF DESCRIPTION OF THE DRAWINGS

Figure 1 illustrates different embodiments of the invention wherein a process to produce a dried carboxylic acid **410** is provided.
Figure 2 illustrates an embodiment of the invention, wherein an purge stream is created. This figure is a detailed illustration on zone **700** in figure 1.

### DETAILED DESCRIPTION

It should be understood that the following is not intended to be an exclusive list of defined terms. Other definitions may be provided in the foregoing description, such as, for example, when accompanying the use of a defined term in context.

As used herein, the terms "a," "an," and "the" mean one or more.

As used herein, the term "and/or," when used in a list of two or more items, means that any one of the listed items can be employed by itself or any combination of two or more of the listed items can be employed. For example, if a composition is described as containing components A, B, and/or C, the composition can contain A alone; B alone; C alone; A and B in combination; A and C in combination, B and C in combination; or A, B, and C in combination.

As used herein, the terms "comprising," "comprises," and "comprise" are open-ended transition terms used to transition from a subject recited before the term to one or more elements recited after the term, where the element or elements listed after the transition term are not necessarily the only elements that make up the subject.

As used herein, the terms "having," "has," and "have" have the same open-ended meaning as "comprising," "comprises," and "comprise" provided above.

As used herein, the terms "including," "includes," and "include" have the same open-ended meaning as "comprising," "comprises," and "comprise" provided above.

The present description uses numerical ranges to quantify certain parameters relating to the invention. It should be understood that when numerical ranges are provided, such ranges are to be construed as providing literal support for claim limitations that only recite the lower value of the range as well as claim limitations that only recite the upper value of the range. For example, a disclosed numerical range of 10 to 100 provides literal support for a claim reciting "greater than 10" (with no upper bounds) and a claim reciting "less than 100" (with no lower bounds).

The present description uses specific numerical values to quantify certain parameters relating to the invention, where the specific numerical values are not expressly part of a numerical range. It should be understood that each specific numerical value provided herein is to be construed as providing literal support for a broad, intermediate, and narrow range. Degrees Fahrenheit (°F) can be converted into degrees Celsius (°C) according to the following equation: °C = (°F - 32) * (5/9). The broad range associated with each specific numerical value is the numerical value plus and minus 60 percent of the numerical value, rounded to two significant digits. The intermediate range associated with each specific numerical value is the numerical value plus and minus 30 percent of the numerical value, rounded to two significant digits. The narrow range associated with each specific numerical value is the numerical value plus and minus 15 percent of the numerical value, rounded to two significant digits. For example, if the specification describes a specific temperature of 62 °F, such a description provides literal support for a broad numerical range of 25 °F to 99 °F (62 °F +/- 37 °F), an intermediate numerical range of 43 °F to 81 °F (62 °F +/- 19 °F), and a narrow numerical range of 53 °F to 71 °F (62 °F +/- 9 °F). These broad, intermediate, and narrow numerical ranges should be applied not only to the specific values, but should also be applied to differences between these specific values. Thus, if the specification describes a first pressure of 110 psia and a second pressure of 48 psia (a difference of 62 psi), the broad, intermediate, and narrow ranges for the pressure difference between these two streams would be 25 to 99 psi, 43 to 81 psi, and 53 to 71 psi, respectively. psi can be converted into pascals (Pa) according to the following equation: 1 psi = 6894.76 pascals (Pa).

The present invention relates to a process for producing a carboxylic acid composition, wherein the process is defined as set out in the claims.

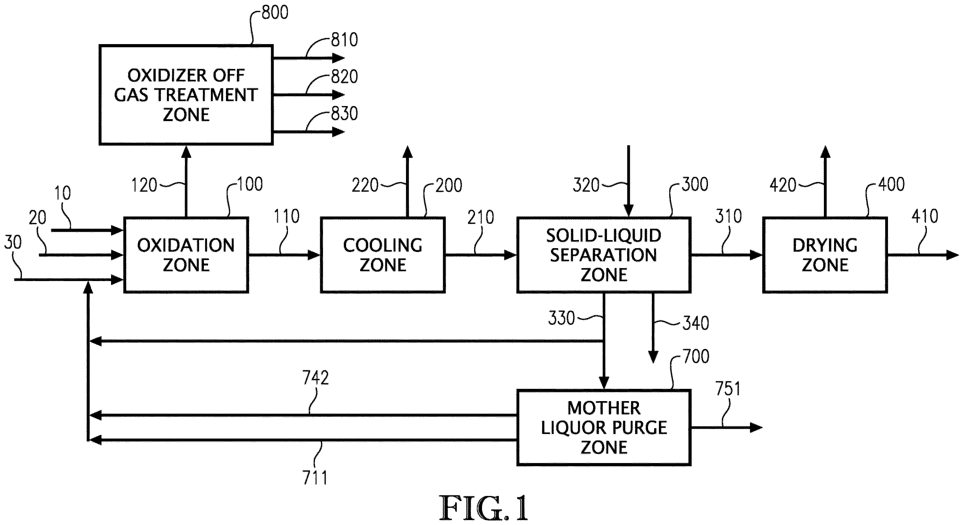

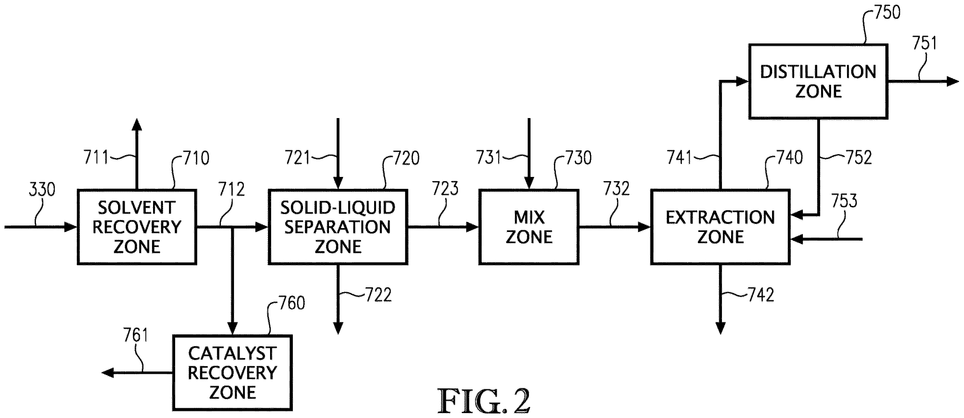

One embodiment of the present invention is illustrated in Figures 1 and 2. The present invention provides a process for recovering a portion of oxidation solvent, a portion of oxidation catalyst, and removing a portion of oxidation by-products and raw material impurities from a solvent stream generated in a process to make furan-2,5-dicarboxylic acid (FDCA). :
**Step (a)** comprises feeding oxidation solvent, a catalyst system, a gas stream comprising oxygen, and oxidizable raw material comprising at least one compound selected from the group of formula: 5-(hydroxymethyl)furfural (5-HMF), 5-(chloromethyl)furfural (5-CMF), 2,5-dimethylfuran (2,5 -DMF), 5-HMF esters (5-R(CO)OCH₂-furfural where R = alkyl, cycloalkyl and aryl), 5-HMF ethers (5-R'OCH₂-furfural, where R' = alkyl, cycloalkyl and aryl), 5-alkyl furfurals (5-R"-furfural, where R" = alkyl, cycloalkyl and aryl), alkylcarboxylate of methyfuran (5-R‴O(CO)-methylfuran, where R‴ = alkyl, cycloalkyl), alkylcarboxylate of alkoxyfuran (5-RʺʺO(CO)-OR‴ʺfuran, where Rʺʺ = alkyl, cycloalkyl and aryl and R‴ʺ = alkyl, cycloalkyl and aryl), mixed feed-stocks of 5-HMF and 5-HMF esters and mixed feed-stocks of 5-HMF and 5-HMF ethers, mixed feed-stocks of 5-HMF and 5-alkyl furfurals, mixed feed-stocks of 5-HMF and 5-CMF, mixed feed-stocks of 5-HMF and 2,5-DMF, mixed feed-stocks of 5-HMF and alkylcarboxylate of methyfuran, mixed feed-stocks of 5-HMF and alkylcarboxylate of alkoxyfuran to an oxidation zone **100** to generate a crude carboxylic acid slurry **110** comprising furan-2,5-dicarboxylic (FDCA). Structures for the preferred oxidizable raw material compounds are outlined below:

### Preferred 5-HMF Derivative Feeds

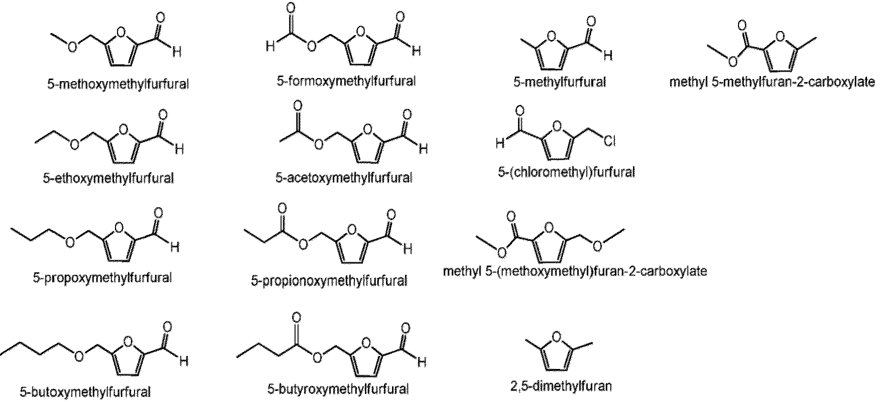

5-HMF feed is oxidized with elemental O₂ in a multi-step reaction to form FDCA with 5-formyl furan-2-carboxyic acid (FFCA) as a key intermediate (Eq 1). Oxidation of 5-(acetoxymethyl)furfural (5-AMF), which contains an oxidizable ester and aldehydes moieties, produces FDCA, FFCA, and acetic acid (Eq 2). Similarly, oxidation of 5-(ethoxymethyl)furfural (5-EMF) produces FDCA, FFCA, 5-(ethoxycarbonyl)furan-2-carboxylic acid (EFCA) and acetic acid (Eq 3).

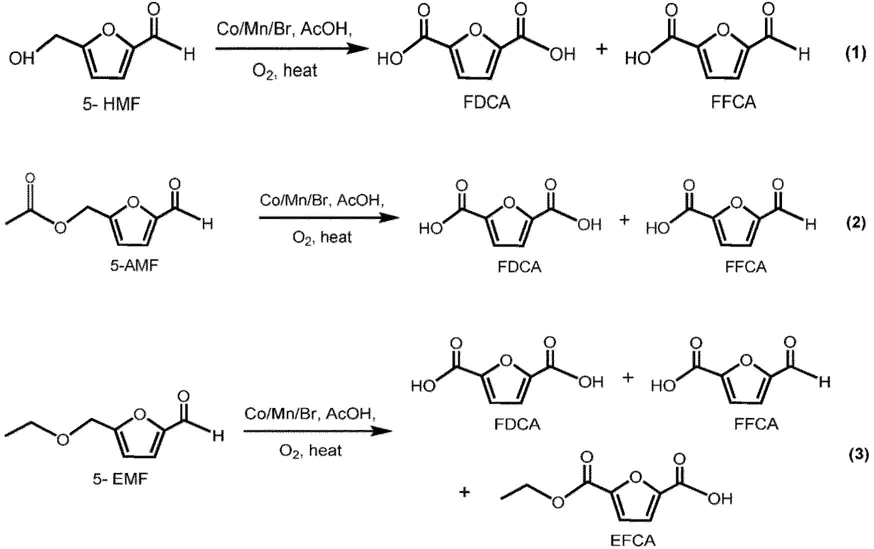

Streams routed to the primary oxidation zone **100** comprise gas stream **10** comprising oxygen, and stream **30** comprising oxidation solvent, and stream **20** comprising oxidizable raw material. In another embodiment, streams routed to the oxidization zone **100** comprise gas stream **10** comprising oxygen and stream **20** comprising oxidation solvent, catalyst, and oxidizable raw material. In yet another embodiment, the oxidation solvent, gas comprising oxygen, catalyst system, and oxidizable raw materials can be fed to the oxidization zone **100** as separate and individual streams or combined in any combination prior to entering the oxidization zone 100 wherein said feed streams may enter at a single location or in multiple locations into oxidizer zone **100.**

Suitable catalyst systems is at least one compound selected from, but are not limited to, cobalt, bromine, and manganese compounds, which are soluble in the selected oxidation solvent. The preferred catalyst system comprises cobalt, manganese and bromine wherein the weight ratio of cobalt to manganese in the reaction mixture is from about 10 to about 400 and the weight ratio of cobalt to bromine is from about 0.7 to about 3.5. Data shown in **Tables 1** to **3** demonstrate that very high yield of FDCA can be obtained using 5-HMF or its derivatives using the catalyst composition described above.

Suitable oxidation solvents include, but are not limited to, aliphatic mono-carboxylic acids, preferably containing 2 to 6 carbon atoms, and mixtures thereof, and mixtures of these compounds with water. In one embodiment, the oxidation solvent comprises acetic acid wherein the weight % of acetic acid in the oxidation solvent is greater than 50%, greater than 75%, greater than 85%, and greater than 90%. In another embodiment, the oxidation solvent comprises acetic acid and water wherein the proportions of acetic acid to water is greater than 1:1, greater than 6:1, greater than 7:1, greater than 8:1, and greater than 9:1.

The temperature in oxidation zone can range from 100°C to 220°C, or 100°C to 200°C, or 130°C to 180°C, or 100°C to 180°C and can preferably range from 110°C to 160°C. In another embodiment, the temperature in oxidation zone can range from 105°C to 140°C.

One advantage of the disclosed oxidation conditions is low carbon burn as illustrated in **Tables 1 to 3.** Oxidizer off gas stream **120** is routed to the oxidizer off gas treatment zone **800** to generate an inert gas stream **810,** liquid stream **820** comprising water, and a recovered oxidation solvent stream **830** comprising condensed solvent. In one embodiment, at least a portion of recovered oxidation solvent stream **830** is routed to wash solvent stream **320** to become a portion of the wash solvent stream **320** for the purpose of washing the solids present in the solid-liquid separation zone. In another embodiment, the inert gas stream **810** can be vented to the atmosphere. In yet another embodiment, at least a portion of the inert gas stream **810** can be used as an inert gas in the process for inerting vessels and or used for convey gas for solids in the process. In another embodiment, at least a portion of the energy in stream **120** is recovered in the form of steam and or electricity.

In another embodiment of the invention, a process is provided for producing furan-2,5-dicarboxylic acid (FDCA) in high yields by liquid phase oxidation that minimizes solvent and starting material loss through carbon burn. The process comprises oxidizing at least one oxidizable compound in an oxidizable raw material stream **30** in the presence of an oxidizing gas stream **10,** oxidation solvent stream **20,** and at least one catalyst system in a oxidation zone **100;** wherein the oxidizable compound is 5-(hydroxymethyl)furfural (5-HMF); wherein the solvent stream comprises acetic acid with or without the presence of water; wherein the catalyst system comprising cobalt, manganese, and bromine, wherein the weight ratio of cobalt to manganese in the reaction mixture is from about 10 to about 400. In this process, the temperature can vary from about 100°C to about 220°C, from about 105°C to about 180°C, and from about 110°C to about 160°C. The cobalt concentration of the catalyst system can range from about 1000 ppm to about 6000 ppm, and the amount of manganese can range from about 2 ppm to about 600 ppm, and the amount of bromine can range from about 300 ppm to about 4500 ppm with respect to the total weight of the liquid in the reaction medium.

**Step (b)** comprises routing the crude carboxylic acid slurry **110** comprising FDCA to cooling zone **200** to generate a cooled crude carboxylic acid slurry stream **210** and a 1^{st} vapor stream **220** comprising oxidation solvent vapor. The cooling of crude carboxylic slurry stream **110** can be accomplished by any means known in the art. Typically, the cooling zone **200** comprises a flash tank. In another embodiment, a portion up to 100% of the crude carboxylic acid slurry stream **110** is routed directly to solid-liquid separation zone **300,** thus said portion up to 100% is not subjected to cooling in cooling zone **200.** The temperature of stream **21**0 can range from 35°C to 210°C, 55°C to 120°C, and preferably from 75°C to 95°C.

**Step (c )** comprises isolating, washing, and dewatering solids present in the cooled crude carboxylic acid slurry stream **210** in the solid-liquid separation zone **300** to generate a crude carboxylic acid wet cake stream **310** comprising FDCA. These functions may be accomplished in a single solid-liquid separation device or multiple solid-liquid separation devices. The solid-liquid separation zone comprises at least one solid-liquid separation device capable of separating solids and liquids, washing solids with a wash solvent stream **320,** and reducing the % moisture in the washed solids to less than 30 weight %, less than 20 weight %, less than 15 weight %, and preferably less than 10 weight %.

Equipment suitable for the solid liquid separation zone can typically be at least one of the following types of devices: centrifuge, cyclone, rotary drum filter, belt filter, pressure leaf filter, candle filter, and the like. The preferred solid liquid separation device for the solid liquid separation zone is a rotary pressure drum filter.

The temperature of cooled crude carboxylic acid slurry steam **210** which is routed to the solid-liquid separation zone **300** can range from 35 °C to 210°C, 55°C to 120°C, and is preferably from 75°C to 95°C. Wash solvent stream **320** comprises a liquid suitable for displacing and washing mother liquor from the solids. In one embodiment, a suitable wash solvent comprises acetic acid. In another embodiment, a suitable wash solvent comprises acetic acid and water. In yet another embodiment, a suitable wash solvent comprises water and can be 100% water. The temperature of the wash solvent can range from 20°C to 160°C, 40°C to 110°C, and preferably from 50 °C to 90°C.

The amount of wash solvent used is defined as the wash ratio and equals the mass of wash divided by the mass of solids on a batch or continuous basis. The wash ratio can range from about 0.3 to about 5, about 0.4 to about 4, and preferably from about 0.5 to 3. After solids are washed in the solid liquid separation zone, they are dewatered. Dewatering involves reducing the mass of moisture present with the solids to less than 30% by weight, less than 25% by weight, less than 20% by weight, and most preferably less than 15% by weight resulting in the generation of a crude carboxylic acid wet cake stream **310** comprising FDCA.

In one embodiment, dewatering is accomplished in a filter by passing a stream comprising gas through the solids to displace free liquid after the solids have been washed with a wash solvent. In an embodiment, dewatering of the wet cake solids in solid-liquid separation zone **300** can be implemented before washing and after washing the wet cake solids in zone **300** to minimize the amount of oxidizer solvent present in the wash liquor stream **340.** In another embodiment, dewatering is achieved by centrifugal forces in a perforated bowl or solid bowl centrifuge.

The mother liquor steam **330** generated in solid-liquid separation zone **300** comprises oxidation solvent, catalyst, and impurities. From 5wt% to 95wt%, from 30wt% to 90wt%, and most preferably from 40wt% to 80wt% of mother liquor present in the crude carboxylic acid slurry **110** is isolated in solid-liquid separation zone **300** to generate mother liquor stream **330** resulting in dissolved matter comprising impurities present in mother liquor stream **330** not going forward in the process.

In one embodiment, a portion of mother liquor stream **330** is routed to a mother liquor purge zone **700,** wherein a portion is at least 5 weight %, at least 25 weight %, at least 45 weight %, at least 55 weight % at least 75 weight %, or at least 90 weight %. In another embodiment, at least a portion of the mother liquor stream **330** is routed back to the oxidation zone **100,** wherein a portion is at least 5 weight %. In yet another embodiment, at least a portion of mother liquor stream **330** is routed to a mother liquor purge zone **700** and to the oxidation zone **100** wherein a portion is at least 5 weight %. In one embodiment, the mother liquor purge zone **700** comprises an evaporative step to separate oxidation solvent from stream **330** by evaporation. Solids can be present in mother liquor stream **330** ranging from about 5 weight % to about 0.5 weight %. In yet another embodiment, any portion of mother liquor stream **330** routed to a mother liquor purge zone is first subjected to a solid liquid separation device to control solids present in stream **330** to less than 1wt%, less than 0.5wt%, less than 0.3wt%, or less than 0.1% by weight. Suitable solid liquid separation equipment comprise a disc stack centrifuge and batch pressure filtration solid liquid separation devices. A preferred solid liquid separation device for this application comprises a batch candle filter.

Wash liquor stream **340** is generated in the solid-liquid separation zone **300** and comprises a portion of the mother liquor present in stream **210** and wash solvent wherein the ratio of mother liquor mass to wash solvent mass is less than 3 and preferably less than 2. In an embodiment, at least a portion of wash liquor stream **340** is routed to oxidation zone **100** wherein a portion is at least 5 weight %. In an embodiment, at least a portion of wash liquor stream is routed to mother liquor purge zone **700** wherein a portion is at least 5 weight %. In another embodiment, at least a portion of wash liquor stream **340** is routed to oxidation zone **100** and mother liquor purge zone **700** wherein a portion is at least 5 weight %.

In another embodiment, at least a portion of the crude carboxylic acid slurry stream **110** up to 100 weight % is routed directly to the solid-liquid separation zone **300,** thus this portion will bypass the cooling zone **200.** In this embodiment, feed to the solid-liquid separation zone **300** comprises at least a portion of the crude carboxylic acid slurry stream **110** and wash solvent stream **320** to generate a crude carboxylic acid wet cake stream **310** comprising FDCA. Solids in the feed slurry are isolated, washed, and dewatered in solid-liquid separation zone **300.** These functions may be accomplished in a single solid-liquid separation device or multiple solid-liquid separation devices. The solid-liquid separation zone comprises at least one solid-liquid separation device capable of separating solids and liquids, washing solids with a wash solvent stream **320,** and reducing the % moisture in the washed solids to less than 30 weight %, less than 20 weight %, less than 15 weight %, and preferably less than 10 weight %. Equipment suitable for the solid liquid separation zone can typically be at least one of the following types of devices: centrifuge, cyclone, rotary drum filter, belt filter, pressure leaf filter, candle filter, and the like. The preferred solid liquid separation device for the solid liquid separation zone **300** is a continuous rotary pressure drum filter. The temperature of the crude carboxylic acid slurry stream, which is routed to the solid-liquid separation zone **300** can range from 40°C to 210°C, 60°C to 170°C , °C and is preferably from 80°C to 160°C. The wash stream **320** comprises a liquid suitable for displacing and washing mother liquor from the solids. In one embodiment, a suitable wash solvent comprises acetic acid and water. In another embodiment, a suitable wash solvent comprises water up to 100% water. The temperature of the wash solvent can range from 20°C to 180°C, 40°C and 150°C, and preferably from 50°C to 130°C. The amount of wash solvent used is defined as the wash ratio and equals the mass of wash divided by the mass of solids on a batch or continuous basis. The wash ratio can range from about 0.3 to about 5, about 0.4 to about 4, and preferably from about 0.5 to 3.

After solids are washed in the solid liquid separation zone, they are dewatered. Dewatering involves reducing the mass of moisture present with the solids to less than 30% by weight, less than 25% by weight, less than 20% by weight, and most preferably less than 15% by weight resulting in the generation of a crude carboxylic acid wet cake stream 310. In one embodiment, dewatering is accomplished in a filter by passing a gas stream through the solids to displace free liquid after the solids have been washed with a wash solvent. In another embodiment, the dewatering of the wet cake in solid-liquid separation zone **300** can be implemented before washing and after washing the solids in zone **300** to minimize the amount of oxidizer solvent present in the wash liquor stream **340** by any method known in the art. In yet another embodiment, dewatering is achieved by centrifugal forces in a perforated bowl or solid bowl centrifuge.

Mother liquor steam **330** generated in the solid-liquid separation zone **300** comprising oxidation solvent, catalyst, and impurities. From 5wt% to 95wt%, from 30wt% to 90wt%, and most preferably from 40wt% to 80wt% of mother liquor present in the crude carboxylic acid slurry stream **110** is isolated in solid-liquid separation zone **300** to generate mother liquor stream **330** resulting in dissolved matter comprising impurities present in mother liquor stream **330** not going forward in the process. In one embodiment, a portion of mother liquor stream **330** is routed to a mother liquor purge zone **700,** wherein a portion is at least 5 weight %, at least 25 weight %, at least 45 weight %, at least 55 weight % at least 75 weight %, or at least 90 weight %. In another embodiment, at least a portion is routed back to the oxidation zone **100,** wherein a portion is at least 5 weight %. In yet another embodiment, at least a portion of mother liquor stream **330** is routed to a mother liquor purge zone and to the oxidation zone **100** wherein a portion is at least 5 weight %. In one embodiment, mother liquor purge zone **700** comprises an evaporative step to separate oxidation solvent from stream **330** by evaporation.

Wash liquor stream **340** is generated in the solid-liquid separation zone **300** and comprises a portion of the mother liquor present in stream **210** and wash solvent wherein the ratio of mother liquor mass to wash solvent mass is less than 3 and preferably less than 2. In an embodiment, at least a portion of wash liquor stream **340** is routed to oxidation zone **100** wherein a portion is at least 5 weight %. In an embodiment, at least a portion of wash liquor stream **340** is routed to mother liquor purge zone **700** wherein a portion is at least 5 weight %. In another embodiment, at least a portion of wash liquor stream is routed to oxidation zone **100** and mother liquor purge zone **700** wherein a portion is at least 5 weight %.

Mother liquor stream **330** comprises oxidation solvent, catalyst, soluble intermediates, and soluble impurities. It is desirable to recycle directly or indirectly at least a portion of the catalyst and oxidation solvent present in mother liquor stream **330** back to oxidation zone **100** wherein a portion is at least 5% by weight, at least 25%, at least 45%, at least 65%, at least 85%, or at least 95%. Direct recycling at least a portion of the catalyst and oxidation solvent present in mother liquor stream **330** comprises directly routing a portion of stream **330** to oxidizer zone **100.** Indirect recycling at least a portion of the catalyst and oxidation solvent present in mother liquor stream **330** to oxidation zone **100** comprises routing at least a portion of stream **330** to at least one intermediate zone wherein stream 330 is treated to generate a stream or multiple streams comprising oxidation solvent and or catalyst that are routed to oxidation zone **100.**

**Step (d)** comprises separating components of mother liquor stream **330** in mother liquor purge zone **700** for recycle to the process while also isolating those components not to be recycled comprising impurities. Impurities in stream **330** can originate from one or multiple sources. In an embodiment of the invention, impurities in stream **330** comprise impurities introduced into the process by feeding streams to oxidation zone **100** that comprise impurities. Mother liquor impurities comprise at least one impurity selected from the following group: 2,5-diformylfuran in an amount ranging from about 5 ppm to 800 ppm, 20 ppm to about 1500 ppm, 100 ppm to about 5000 ppm, 150 ppm to about 2.0 wt%; levulinic acid in an amount ranging from about 5 ppm to 800 ppm, 20 ppm to about 1500 ppm, 100 ppm to about 5000 ppm, 150 ppm to about 2.0 wt%; succinic acid in an amount ranging from about 5 ppm to 800 ppm, 20 ppm to about 1500 ppm, 100 ppm to about 5000 ppm, 150 ppm to about 2.0 wt%; acetoxy acetic acid in an amount ranging from about 5 ppm to 800 ppm, 20 ppm to about 1500 ppm, 100 ppm to about 5000 ppm, 150 ppm to about 2.0 wt%

An impurity is defined as any molecule not required for the proper operation of oxidation zone **100.** For example, oxidation solvent, a catalyst system, a gas comprising oxygen, and oxidizable raw material comprising at least one compound selected from the group of formula: 5-(hydroxymethyl)furfural (5-HMF), 5-(chloromethyl)furfural (5-CMF), 2,5-dimethylfuran (2,5 -DMF), 5-HMF esters (5-R(CO)OCH₂-furfural where R = alkyl, cycloalkyl and aryl), 5-HMF ethers (5-R'OCH₂-furfural, where R' = alkyl, cycloalkyl and aryl), 5-alkyl furfurals (5-R"-furfural, where R" = alkyl, cycloalkyl and aryl), alkylcarboxylate of methyfuran (5-R‴O(CO)-methylfuran, where R‴ = alkyl, cycloalkyl), alkylcarboxylate of alkoxyfuran (5-RʺʺO(CO)-OR‴ʺfuran, where Rʺʺ = alkyl, cycloalkyl and aryl and R‴ʺ = alkyl, cycloalkyl and aryl), mixed feed-stocks of 5-HMF and 5-HMF esters and mixed feed-stocks of 5-HMF and 5-HMF ethers, mixed feed-stocks of 5-HMF and 5-alkyl furfurals, mixed feed-stocks of 5-HMF and 5-CMF, mixed feed-stocks of 5-HMF and 2,5-DMF, mixed feed-stocks of 5-HMF and alkylcarboxylate of methyfuran, mixed feed-stocks of 5-HMF and alkylcarboxylate of alkoxyfuran are molecules required for the proper operation of oxidation zone **100** and are not considered impurities. Also, chemical intermediates formed in oxidation zone **100** that lead to or contribute to chemical reactions that lead to desired products are not considered impurities. Oxidation by-products that do not lead to desired products are defined as impurities. Impurities may enter oxidation zone **100** through recycle streams routed to the oxidation zone **100** or by impure raw material streams fed to oxidation zone **100.**

In one embodiment, it is desirable to isolate a portion of the impurities from oxidizer mother liquor stream **330** and purge or remove them from the process as purge stream **751.** In an embodiment of the invention, from 5 to 100% by weight, of mother liquor stream **330** generated in solid-liquid separation zone **300** is routed to mother liquor purge zone **700** wherein a portion of the impurities present in stream **330** are isolated and exit the process as purge stream **751.** The portion of stream **330** going to the mother liquor purge zone **700** can be 5% by weight or greater, 25% by weight or greater, 45% by weight or greater, 65% by weight or greater, 85% by weight or greater, or 95% by weight or greater. Recycle oxidation solvent stream **711** comprises oxidation solvent isolated from stream **330** and can be recycled to the process. The raffinate stream **742** comprises oxidation catalyst isolated from stream 330 which can optionally be recycled to the process. In one embodiment, the raffinate stream **742** is recycled to oxidation zone **100** and contains greater than 30wt%, greater than 50wt%, greater than 80wt%, or greater than 90wt% of the catalyst that entered the mother liquor purge zone **700** in stream **330.** In another embodiment, at least a portion of mother liquor stream **330** is routed directly to oxidation zone **100** without first being treated in mother liquor purge zone **700.** In one embodiment, mother liquor purge zone 700 comprises an evaporative step to separate oxidation solvent from stream **330** by evaporation.

One embodiment of mother liquor purge zone **700** comprises routing at least a portion of oxidizer mother liquor stream **330** to solvent recovery zone **710** to generate a recycle oxidation solvent stream **711** comprising oxidation solvent and an impurity rich waste stream **712** comprising oxidation by products and catalyst. Any technology known in the art capable of separating a volatile solvent from stream **330** may be used. Examples of suitable unit operations include, but are not limited to, batch and continuous evaporation equipment operating above atmospheric pressure, at atmospheric pressure, or under vacuum. A single or multiple evaporative steps may be used. In an embodiment of the invention, sufficient oxidation solvent is evaporated from stream **330** to result in stream **712** being present as a slurry having a weight percent solids greater than 10 weight percent, 20 weight percent, 30 weight percent, 40 weight percent, or 50 weight percent. At least a portion of impurity rich stream **712** can be routed to catalyst recovery zone **760** to generate catalyst rich stream **761.** Examples of suitable unit operations for catalyst recovery zone **760** include, but are not limited to, incineration or burning of the stream to recover noncombustible metal catalyst in stream **761.**

Another embodiment of mother liquor purge zone **700** comprises routing at least a portion of mother liquor stream **330** to solvent recovery zone 710 to generate a recycle oxidation solvent stream **711** comprising oxidation solvent and an impurity rich waste stream **712** comprising oxidation by products and catalyst. Any technology known in the art capable of separating a volatile solvent from stream **330** may be used. Examples of suitable unit operations include but are not limited to batch and continuous evaporation equipment operating above atmospheric pressure, at atmospheric pressure, or under vacuum. A single or multiple evaporative steps may be used. Sufficient oxidation solvent is evaporated from stream **330** to result in impurity rich waste stream **712** being present as slurry with weight % solids greater than 5 weight percent, 10 weight percent, 20 weight percent, and 30 weight percent. At least a portion of the impurity rich waste stream **712** is routed to a solid liquid separation zone **720** to generate a purge mother liquor stream **723** and a wet cake stream **722** comprising impurities. In another embodiment of the invention, all of stream **712** is routed to the solid liquid separation zone **720.** Stream **722** may be removed from the process as a waste stream. Wash stream **721** may also be routed to solid-liquid separation zone **720** which will result in wash liquor being present in stream **723.** It should be noted that zone **720** is a separate and different zone from zone **300.**

Any technology known in the art capable of separating solids from slurry may be used. Examples of suitable unit operations include, but are not limited to, batch or continuous filters, batch or continuous centrifuges, filter press, vacuum belt filter, vacuum drum filter, continuous pressure drum filter, candle filters, leaf filters, disc centrifuges, decanter centrifuges, basket centrifuges, and the like. A continuous pressure drum filter is a preferred device for solid-liquid separation zone **720.**

Purge mother liquor stream **723** comprising catalyst and impurities, and stream **731** comprising a catalyst solvent are routed to mix zone **731** to allow sufficient mixing to generate extraction feed stream **732.** In one embodiment, stream **731** comprises water. Mixing is allowed to occur for at least 30 seconds, 5 minutes, 15 minutes, 30 minutes, or 1 hour. Any technology know in the art may be used for this mixing operation including inline static mixers, continuous stirred tank, mixers, high shear in line mechanical mixers and the like.

Extraction feed stream **732,** recycle extraction solvent stream **752,** and fresh extraction solvent stream **753** are routed to liquid-liquid extraction zone **740** to generate an extract stream **741** comprising impurities and extract solvent, and a raffinate stream **742** comprising catalyst solvent and oxidation catalyst that can be recycled directly or indirectly to the oxidation zone **100.** Liquid-liquid extraction zone **740** may be accomplished in a single or multiple extraction units. The extraction units can be batch and or continuous. An example of suitable equipment for extraction zone **740** includes multiple single stage extraction units. Another example of suitable equipment for extraction zone **740** is a single multi stage liquid-liquid continuous extraction column. Extract stream **741** is routed to distillation zone 750 where extraction solvent is isolated by evaporation and condensation to generate recycle extract solvent stream **752.** The purge stream **751** is also generated and can be removed from the process as a waste purge stream. Batch or continuous distillation may be used in distillation zone **750.**

In another embodiment, the source for oxidizer mother liquor stream **330** feeding mother liquor purge zone **700** may originate from any mother liquor stream comprising oxidation solvent, oxidation catalyst, and impurities generated in process to make furan-2,5-dicarboxylic acid (FDCA). For example, a solvent swap zone downstream of oxidation zone **100** that isolates at least a portion of the FDCA oxidation solvent from stream **110** can be a source for stream 330. Suitable equipment for a solvent swap zone comprises solid-liquid separation devices including centrifuges and filters. Examples of suitable equipment for the solvent swap include, but is not limited to, a disc stack centrifuge or a continuous pressure drum filter.

Terephthalic acid and/or Isophthalic acid is commercially produced by oxidation of paraxylene in the presence of a catalyst, such as, for example, Co, Mn, Br and a solvent. Terephthalic acid used in the production of polyester fibers, films, and resins must be further treated to remove impurities present due to the oxidation of para-xylene. Typical commercial process produce a crude terephthalic acid then dissolve the solid crude terephthalic acid in water at high temperatures and pressures, hydrogenate the resultant solution, cool and crystallize the terephthalic acid product out of solution, and separate the solid terephthalic product from the liquid as discussed in U.S. Patent No. 3,584,039.

Crude terephthalic acid is conventionally made via the liquid phase air oxidation of paraxylene in the presence of a suitable oxidation catalyst. Suitable catalysts comprises at least one selected from, but are not limited to, cobalt, bromine and manganese compounds, which are soluble in the selected solvent. Suitable solvents include, but are not limited to, aliphatic mono-carboxylic acids, preferably containing 2 to 6 carbon atoms, or benzoic acid and mixtures thereof and mixtures of these compounds with water. Preferably the solvent is acetic acid mixed with water, in a ratio of about 5:1 to about 25:1, preferably between about 8:1 and about 20:1. Throughout the specification acetic acid will be referred to as the solvent. However, it should be appreciated that other suitable solvents, such as those disclosed previously, may also be utilized. Patents disclosing the production of terephthalic acid are U.S patent #4,158,738 and #3,996,271.

A number of processes for producing the purified terephthalic acid solid have been developed and are commercially available. Usually, the purified terephthalic acid solid is produced in a multi-step process wherein a crude terephthalic acid is produced. The crude terephthalic acid does not have sufficient quality for direct use as starting material in commercial polyethylene terephthalate(PET). Instead, the crude terephthalic acid is usually refined to purified terephthalic acid solid.

Liquid phase oxidation of p-xylene produces crude terephthalic acid. The crude terephthalic acid is dissolved in water and hydrogenated for the purpose of converting 4-carboxybenzaldehyde to p-toluic acid, which is a more water-soluble derivative, and for the purpose of converting characteristically yellow compounds to colorless derivatives. Significant 4-carboxybenzaldehyde and p-toluic acid in the final purified terephthalic acid product is particularly detrimental to polymerization processes as they may act as chain terminators during the condensation reaction between terephthalic acid and ethylene glycol in the production of PET. Typical purified terephthalic acid contains on a weight basis less than 250 parts per million (ppm) 4-carboxybenzaldehyde and less than 150 ppm p-toluic acid.

The crude terephthalic acid typically contains on a weight basis from about 800 to 7,000 parts per million (ppm) 4-carboxybenzaldehyde and about 200 to 1,500 ppm p-toluic acid as the main impurities. The crude terephthalic acid also contains lesser amounts, about 20-200 ppm range, of aromatic compounds having the structures derived from benzil, fluorenone, and/or anthraquinone, which are characteristically yellow compounds as impurities resulting from coupling side reactions occurring during oxidation of p-xylene

Such a purification process typically comprises adding water to the crude terephthalic acid to form a crude terephthalic acid slurry, which is heated to dissolve the crude terephthalic acid. The crude terephthalic acid solution is then passed to a reactor zone in which the solution is contacted with hydrogen in the presence of a heterogeneous catalyst at temperatures of about 200° to about 375° C. This reduction step converts the various color causing compounds present in the crude terephthalic acid to colorless derivatives. The principal impurity, 4-carboxybenzaldehyde, is converted to p-toluic acid.

Typical crude terephthalic acid contains excessive amounts of both 4-carboxybenzaldehyde and p-toluic acid on a weight basis. Therefore, to achieve less than 250 ppmw 4-carboxybenzaldehyde and less than 150 ppmw p-toluic acid in the purified terephthalic acid requires mechanisms for purifying the crude terephthalic acid and removing the contaminants.

In many processes, colored impurities are hydrogenated to colorless derivatives and leave the process with the terephthalic acid solid product and waste water streams. However, one embodiment of this invention provides an attractive process to produce a purified carboxylic acid slurry by utilizing a solid-liquid displacement zone comprising a solid-liquid separator after oxidation of a crude carboxylic acid slurry product and prior to final filtration and drying without the use of an hydrogenation step.

In another embodiment of the invention the oxidation zone to produce furandicarboxylic acid can comprises at least one oxidizer previously used for a terephthalic acid(TPA) and/or and isophthalic acid(IPA) process. These processes can be any TPA or IPA process known in the art. Examples have been given above but any oxidizer from any TPA and/or IPA process could be used.

### EXAMPLES

This invention can be further illustrated by the following examples of embodiments thereof, although it will be understood that these examples are included merely for the purposes of illustration and are not intended to limit the scope of the invention unless otherwise specifically indicated.

Air oxidation of 5-HMF/5-AMF/5-EMF using cobalt, manganese and ionic bromine catalysts system in acetic acid solvent were conducted. After reaction the heterogeneous mixture was filtered to isolate the crude FDCA. The crude FDCA was washed with acetic acid two times and then twice with DI water. The washed crude FDCA was oven dried at 110 °C under vacuum overnight. The solid and the filtrate were analyzed by Gas Chromatography using BSTFA derivatization method. b* of the solid was measured using a Hunter Ultrascan XE instrument. As shown in **Tables 1 to 3** we have discovered conditions that to generate yields of FDCA up to 89.4%, b* < 6, and low carbon burn (<0.00072 mol/min CO+CO₂)

**Table 1. Results from semi-batch reactions using 5-HMF feed.***

| **Note-Book No.** | **Example** | **Bromide source** | **Co conc (ppm)** | **Mn conc (ppm)** | **Br conc (ppm)** | **yield of FDCA (%)** | **yield of FFCA (%)** | **CO (total mol)** | **CO₂ (total mol)** | **CO_{X} (mol/min)** | **color (b*)** |
|---|---|---|---|---|---|---|---|---|---|---|---|
| Ex-000250-187 | 1a | solid NaBr | 2000 | 93.3 | 3000 | 81.6 | 0.81 | 0.013 | 0.078 | 0.000758 | 13.91 |
| Ex-000186-019 | 1b | solid NaBr | 2000 | 93.3 | 3000 | 82.6 | 0.87 | 0.013 | 0.092 | 0.000875 | 14.14 |
| Ex-000186-004 | 2a | aqueous HBr | 2000 | 93.3 | 3000 | 89.4 | 0.58 | 0.003 | 0.061 | 0.000533 | 5.845 |
| Ex-000186-016 | 2b | aqueous HBr | 2000 | 93.3 | 3000 | 88.6 | 0.8 | 0.0037 | 0.061 | 0.000539 | 6.175 |

| | | | | | | | | | | | |
|---|---|---|---|---|---|---|---|---|---|---|---|
| *P= 130 psig, COₓ (mol/min) = CO (mol/min) + CO2 (mol/min). | | | | | | | | | | | |

**Table 2. Results from semi-batch reactions using 5-AMF feed.***

| **NoteBook #** | **Example** | **Co conc (ppmw)** | **Mn conc (PPmw)** | **Br conc (PPmw)** | **Temperature (°C)** | **% yield of FDCA** | **% yield of FFCA** | **CO (total mol)** | **CO2 (total mol)** | **COₓ(mol/min)** | **color(b*)** |
|---|---|---|---|---|---|---|---|---|---|---|---|
| 186-026 | 2a | 2500 | 116.8 | 2500 | 130 | 88.2 | 0.25 | 0.0052 | 0.08 | 0.00071 | 4.4 |
| 186-044 | 2b | 2000 | 93.5 | 3000 | 130 | 90.2 | 0.16 | 0.005 | 0.046 | 0.000425 | 6.8 |

| | | | | | | | | | | | |
|---|---|---|---|---|---|---|---|---|---|---|---|
| *P= 130 psig, COₓ (mol/min) = CO (mol/min) + CO2 (mol/min). | | | | | | | | | | | |

**Table 3. Results from semi-batch reactions using 5-EMF feed.***

| **NoteBook #** | **Example** | **Co conc (ppmw)** | **Mn conc (ppmw)** | **Br conc (ppmw)** | **Temperatur e (°C)** | **%yield of FDCA** | **% yield of FFCA** | **%yield of EFCA** | **CO (total mol)** | **CO2 (total mol)** | **COₓ(mol/m in)** | **color (b*)** |
|---|---|---|---|---|---|---|---|---|---|---|---|---|
| 186-028 | 3a | 2500 | 116.8 | 2500 | 130 | 88.8 | 0.02 | 0.225 | 0.008 | 0.068 | 0.00063333 | 3.97 |
| 186-031 | 3b | 2000 | 93.5 | 3000 | 130 | 88.0 | 0.09 | 0.43 | 0.008 | 0.078 | 0.00071667 | 2.48 |
| 186-034 | 3c | 2000 | 93.5 | 3000 | 105 | 86.0 | 2.92 | 1.4 | 0.005 | 0.046 | 0.000425 | 6.66 |
| 186-038 | 3d | 2500 | 116.8 | 2500 | 130 | 87.4 | 0.42 | 1.3 | 0.009 | 0.064 | 0.00060833 | 2.74 |

| | | | | | | | | | | | | |
|---|---|---|---|---|---|---|---|---|---|---|---|---|
| *P= 130 psig, COₓ (mol/min) = CO (mol/min) + CO2 (mol/min). | | | | | | | | | | | | |

## Claims

1. A process for producing a carboxylic acid composition, said process comprising:
oxidizing in a primary oxidation zone, at least one oxidizable compound comprising at least one selected from the group consisting of 5-(hydroxymethyl)furfural (5-HMF), 5-(chloromethyl)furfural (5-CMF), 2,5-dimethylfuran (2,5 -DMF), 5-HMF esters (5-R(CO)OCH₂-furfural where R = alkyl, cycloalkyl and aryl), 5-HMF ethers (5-R'OCH₂-furfural, where R' = alkyl, cycloalkyl and aryl), 5-alkyl furfurals (5-R"-furfural, where R" = alkyl, cycloalkyl and aryl), alkylcarboxylate of methylfuran (5-R‴O(CO)-methylfuran, where R‴ = alkyl, cycloalkyl), alkylcarboxylate of alkoxyfuran (5-RʺʺO(CO)-OR‴ʺfuran, where Rʺʺ = alkyl, cycloalkyl and aryl and R‴ʺ = alkyl, cycloalkyl and aryl), mixed feed-stocks of 5-HMF and 5-HMF esters and mixed feed-stocks of 5-HMF and 5-HMF ethers, mixed feed-stocks of 5-HMF and 5-alkyl furfurals, mixed feed-stocks of 5-HMF and 5-CMF, mixed feed-stocks of 5-HMF and 2,5-DMF, mixed feed-stocks of 5-HMF and alkylcarboxylate of methylfuran mixed feed-stocks of 5-HMF and alkylcarboxylate of alkoxyfuran, in the presence of a solvent stream, an oxidizing gas, and a catalyst system at a temperature of about 110°C to about 220°C to produce said carboxylic acid composition comprising furandicarboxylic acid at a yield of 80% or more; wherein said primary oxidation zone comprises at least one oxidation reactor, wherein said at least one oxidation reactor in said oxidation zone has been previously used in an terephthalic acid and/or isophthalic acid process;
generating a cooled crude carboxylic acid slurry stream;
isolating, washing, and dewatering solids present in the cooled crude carboxylic acid slurry stream in a solid-liquid separation zone to generate (i) a crude carboxylic acid wet cake stream comprising FDCA and (ii) a mother liquor stream comprising oxidation solvent, catalyst, and impurities,
wherein from 5 wt.% to 95 wt.% of mother liquor present in the crude carboxylic acid slurry is isolated in the solid-liquid separation zone to generate the mother liquor stream, and
wherein the solid-liquid separation zone comprises at least one solid-liquid separation device capable of separating solids and liquids, washing solids with a wash solvent stream, and reducing the % moisture in the washed solids to less than 30 wt.%;
routing a portion of the mother liquor stream to a mother liquor purge zone, wherein a portion is at least 5 wt.%, and wherein the mother liquor purge zone comprises an evaporative step to separate oxidation solvent from the mother liquor stream by evaporation.

2. A process according to claim 1 wherein said oxidation reactor comprises a bubble column.

3. A process according to claim 2 wherein said catalyst comprises cobalt in a range from about 700 ppm to about 4500 ppm by weight with respect to the weight of the liquid in the primary oxidation zone, manganese in an amount ranging from about 20 ppm by weight to about 400 ppm by weight with respect to the weight of the liquid in the primary oxidation zone and bromine in an amount ranging from about 700 ppm by weight to about 4000 ppm by weight with respect to the weight of the liquid in the primary oxidation zone.

4. A process according to claim 1 wherein said catalyst comprises cobalt in a range from about 1000 ppm to about 4000 ppm with respect to the weight of the liquid in the primary oxidation zone, manganese in an amount ranging from about 20 ppm to about 200 ppm by weight with respect to the weight of the liquid in the primary oxidation zone and bromine in an amount ranging from about 1000 ppm to about 4000 ppm by weight with respect to the weight of the liquid in the primary oxidation zone.

5. A process according to claim 1 wherein said carboxylic acid composition comprises at least one carboxylic acid selected from the group consisting of FDCA and FFCA.

6. A process according to claim 1 wherein said carboxylic acid composition comprises FDCA.

7. A process according to claim 1 wherein said oxidizing is conducted at a temperature from about 110°C to about 180°C.

8. A process according to claim 2 wherein said oxidizing is conducted at a temperature from about 110°C to about 160°C.

9. A process according to claim 1 wherein said oxidizing is conducted at a pressure from about 50 psig to about 1000 psig.

10. A process according to claim 1 wherein said oxidizing is conducted at a pressure from about 50 psig to about 400 psig.

11. A process according to claim 1 wherein said oxidizable raw material stream pH ranges from about -4.0 to about 1.0.

12. A process according to claim 1 wherein said oxidizable raw material stream pH ranges from about -1.8 to about 1.0.

13. A process according to claim 1 wherein said oxidizable raw material stream pH ranges from about -1.5 to about 1.0.

## Patentansprüche

1. Ein Verfahren zum Herstellen einer Carbonsäure Zusammensetzung, besagtes Verfahren aufweisend:
Oxidieren in einem ersten Oxidationsbereich mindestens einer oxidierbaren Verbindung aufweisend mindestens eine ausgewählt aus der Gruppe bestehend aus 5-(Hydroxymethyl)furfural (5-HMF), 5-(Chlormethyl)furfural (5-CMF), 2,5-Dimethylfuran (2,5-DMF), 5-HMF Ester (5-R(CO)OCH₂-Furfural, wobei R = Alkyl, Cycloalkyl und Aryl), 5-HMF Ether (5-R'OCH₂-Furfural, wobei R' = Alkyl, Cycloalkyl und Aryl), 5-Alkylfurfurale (5-R"-Furfural, wobei R" = Alkyl, Cycloalkyl und Aryl), Alkylcarboxylate von Methylfuran (5-R‴O(CO)-Methylfuran, wobei R‴ = Alkyl, Cycloalkyl), Alkylcarboxylate von Alkoxyfuran (5-RʺʺO(CO)-OR‴ʺfuran, wobei Rʺʺ =Alkyl, Cycloalkyl und Aryl und R‴ʺ = Alkyl, Cycloalkyl und Aryl), gemischte Ausgangsmaterialen aus 5-HMF und 5-HMF Estern und gemischte Ausgangsmaterialien aus 5-HMF und 5-HMF Ethern, gemischte Ausgangsmaterialien aus 5-HMF und 5-Alkylfurfuralen, gemischte Ausgangsmaterialien aus 5-HMF und 5-CMF, gemischte Ausgangsmaterialien aus 5-HMF und 2,5-DMF, gemischte Ausgangsmaterialien aus 5-HMF und Alkylcarboxylaten von Methylfuran, gemischte Ausgangsmaterialien aus 5-HMF und Alkylcarboxylaten von Alkoxyfuran, in Anwesenheit eines Lösungsmittelstroms, eines oxidierenden Gases und eines Katalysatorsystems bei einer Temperatur von etwa 110°C bis etwa 220°C zum Herstellen besagter Carbonsäure Zusammensetzung aufweisend Furandicarbonsäure in einer Ausbeute von 80% oder mehr; wobei besagter erster Oxidationsbereich mindestens einen Oxidationsreaktor aufweist, wobei der besagte mindestens eine Oxidationsreaktor in besagtem Oxidationsbereich zuvor in einem Terephthalsäure und/oder Isophthalsäure Verfahren verwendet wurde;
Erzeugen eines gekühlten rohen Carbonsäure Schlammstroms;
Isolieren, Waschen und Entwässern von Feststoffen vorhanden in dem gekühlten rohen Carbonsäure Schlammstrom in einem fest-flüssig Trennbereich zum Erzeugen (i) eines rohen Carbonsäure Feuchtkuchenstroms aufweisend FDCA und (ii) eines Mutterlaugenstroms aufweisend Oxidationslösungsmittel, Katalysator und Unreinheiten,
wobei von 5 Gew.-% bis 95 Gew.-% der Mutterlauge vorhanden in dem rohen Carbonsäure-Schlamm isoliert wird in dem fest-flüssig Trennbereich zum Generieren des Mutterlaugenstroms, und
wobei der fest-flüssig Trennbereich mindestens ein fest-flüssig Trenngerät aufweist fähig zum Trennen von Feststoffen und Flüssigkeiten, zum Waschen von Feststoffen mit einem Waschlösungsmittelstrom, und zum Reduzieren der % Feuchtigkeit in den gewaschenen Feststoffen zu weniger als 30 Gew.%;
Zuleiten eines Teils des Mutterlaugenstroms in einen Mutterlaugenreinigungsbereich, wobei ein Teil mindestens 5 Gew.% ist, und wobei der Mutterlaugenreinigungsbereich einen Evaporationsschritt zum Abtrennen des Oxidationslösungsmittels von der Mutterlauge durch Evaporation aufweist.

2. Verfahren nach Anspruch 1, wobei besagter Oxidationsreaktor eine Blasenkolonne aufweist.

3. Verfahren nach Anspruch 2, wobei besagter Katalysator Cobalt in einem Bereich von etwa 700 ppm bis etwa 4500 ppm nach Gewicht im Verhältnis zum Gewicht der Flüssigkeit in dem ersten Oxidationsbereich, Mangan in einer Menge reichend von etwa 20 ppm nach Gewicht bis etwa 400 ppm nach Gewicht im Verhältnis zum Gewicht der Flüssigkeit in dem ersten Oxidationsbereich und Brom in einer Menge reichend von etwa 700 ppm nach Gewicht bis etwa 4000 ppm nach Gewicht im Verhältnis zu dem Gewicht der Flüssigkeit in dem ersten Oxidationsbereich aufweist.

4. Verfahren nach Anspruch 1, wobei besagter Katalysator Cobalt in einem Bereich von etwa 1000 ppm bis etwa 4000 ppm nach Gewicht im Verhältnis zum Gewicht der Flüssigkeit in dem ersten Oxidationsbereich, Mangan in einer Menge reichend von etwa 20 ppm nach Gewicht bis etwa 200 ppm nach Gewicht im Verhältnis zum Gewicht der Flüssigkeit in dem ersten Oxidationsbereich und Brom in einer Menge reichend von etwa 1000 ppm nach Gewicht bis etwa 4000 ppm nach Gewicht im Verhältnis zu dem Gewicht der Flüssigkeit in dem ersten Oxidationsbereich aufweist.

5. Verfahren nach Anspruch 1, wobei besagte Carbonsäure Zusammensetzung mindestens eine Carbonsäure aufweist, ausgewählt aus der Gruppe bestehend aus FDCA und FFCA.

6. Verfahren nach Anspruch 1, wobei besagte Carbonsäure Zusammensetzung FDCA aufweist.

7. Verfahren nach Anspruch 1, wobei besagtes Oxidieren durchgeführt wird bei einer Temperatur von etwa 110 °C bis etwa 180 °C.

8. Verfahren nach Anspruch 2, wobei besagtes Oxidieren durchgeführt wird bei einer Temperatur von etwa 110 °C bis etwa 160 °C.

9. Verfahren nach Anspruch 1, wobei besagtes Oxidieren durchgeführt wird bei einem Druck von etwa 50 psig bis etwa 1000 psig.

10. Verfahren nach Anspruch 1, wobei besagtes Oxidieren durchgeführt wird bei einem Druck von etwa 50 psig bis etwa 400 psig.

11. Verfahren nach Anspruch 1, wobei der pH des besagten oxidierbaren Rohmaterialstroms von - 4,0 bis etwa 1,0 reicht.

12. Verfahren nach Anspruch 1, wobei der pH des besagten oxidierbaren Rohmaterialstroms von - 1,8 bis etwa 1,0 reicht.

13. Verfahren nach Anspruch 1, wobei der pH des besagten oxidierbaren Rohmaterialstroms von - 1,5 bis etwa 1,0 reicht.

## Revendications

1. Procédé pour produire une composition d'acide carboxylique, ledit procédé comprenant :
l'oxydation, dans une zone d'oxydation primaire, d'au moins un composé oxydable comprenant au moins l'un choisi dans le groupe constitué par le 5-(hydroxyméthyl)furfural (5-HMF), le 5-(chlorométhyl)furfural (5-CMF), le 2,5-diméthylfurane (2,5-DMF), les esters de 5-HMF (5-R(CO)OCH₂-furfural où R = alkyle, cycloalkyle et aryle), les éthers de 5-HMF (5-R'OCH₂-furfural où R' = alkyle, cycloalkyle et aryle), les 5-alkyl-furfurals (5-R"-furfural où R" = alkyle, cycloalkyle et aryle), un alcoxycarboxylate de méthylfurane (5-R‴O(CO)-méthylfurane où R‴ = alkyle, cycloalkyle), un alkylcarboxylate d'alcoxyfurane (5-R""O(CO)-OR‴ʺ-furane où R"" = alkyle, cycloalkyle et aryle et R‴ʺ = alkyle, cycloalkyle et aryle), les charges mixtes de 5-HMF et d'esters de 5-HMF et les charges mixtes de 5-HMF et d'éthers de 5-HMF, les charges mixtes de 5-HMF et de 5-alkyl-furfurals, les charges mixtes de 5-HMF et de 5-CMF, les charges mixtes de 5-HMF et de 2,5-DMF, les charges mixtes de 5-HMF et d'alkylcarboxylate de méthylfurane, les charges mixtes de 5-HMF et d'alkylcarboxylate d'alcoxyfurane, en présence d'un courant de solvant, d'un gaz oxydant et d'un système catalyseur à une température d'environ 110°C à environ 220°C pour produire ladite composition d'acide carboxylique comprenant de l'acide furanedicarboxylique avec un rendement de 80 % ou plus ; dans laquelle ladite zone d'oxydation primaire comprend au moins un réacteur d'oxydation, dans laquelle ledit au moins un réacteur d'oxydation dans ladite zone d'oxydation a été utilisé antérieurement dans un procédé d'acide téréphtalique et/ou d'acide isophtalique ;
la génération d'un courant de bouillie d'acide carboxylique brut refroidi ;
l'isolation, le lavage et la déshydratation des solides présents dans l'acide carboxylique brut refroidi dans une zone de séparation de solides-liquides pour générer (i) un courant de gâteau humide d'acide carboxylique brut comprenant du FDCA et (ii) un courant de liqueur mère comprenant du solvant d'oxydation, du catalyseur, et des impuretés,
dans lesquels 5 % en poids à 95 % en poids de la liqueur mère présente dans la bouillie d'acide carboxylique brut sont isolés dans la zone de séparation de solides-liquides pour générer le courant de liqueur mère, et
dans lesquels la zone de séparation de solides-liquides comprend au moins un dispositif de séparation de solides-liquides capable de séparer des solides et des liquides, de laver les solides avec un courant de solvant de lavage, et de réduire le pourcentage d'humidité dans les solides lavés à moins de 30 % en poids ;
l'acheminement d'une portion du courant de liqueur mère jusqu'à une zone de purge de liqueur mère, dans lequel la portion est d'au moins 5 % en poids, et dans lequel la zone de purge de liqueur mère comprend une étape d'évaporation pour séparer le solvant d'oxydation d'avec le courant de liqueur mère par évaporation.

2. Procédé selon la revendication 1, dans lequel ledit réacteur d'oxydation comprend une colonne à bulles.

3. Procédé selon la revendication 2, dans lequel ledit catalyseur comprend du cobalt à raison d'environ 700 ppm à environ 4500 ppm en poids par rapport au poids du liquide dans la zone d'oxydation primaire, du manganèse en une quantité située dans la plage allant d'environ 20 ppm en poids à environ 400 ppm en poids par rapport au poids du liquide dans la zone d'oxydation primaire, et du brome en une quantité située dans la plage allant d'environ 700 ppm en poids à environ 4000 ppm en poids par rapport au poids du liquide dans la zone d'oxydation primaire.

4. Procédé selon la revendication 1, dans lequel ledit catalyseur comprend du cobalt à raison d'environ 1000 ppm à environ 4000 ppm par rapport au poids du liquide dans la zone d'oxydation primaire, du manganèse en une quantité située dans la plage allant d'environ 20 ppm en poids à environ 200 ppm en poids par rapport au poids du liquide dans la zone d'oxydation primaire, et du brome en une quantité située dans la plage allant d'environ 1000 ppm en poids à environ 4000 ppm en poids par rapport au poids du liquide dans la zone d'oxydation primaire.

5. Procédé selon la revendication 1, dans lequel ladite composition d'acide carboxylique comprend au moins un acide carboxylique choisi dans le groupe constitué par le FDCA et le FFCA.

6. Procédé selon la revendication 1, dans lequel ladite composition d'acide carboxylique comprend du FDCA.

7. Procédé selon la revendication 1, dans lequel ladite oxydation est effectuée à une température d'environ 110°C à environ 180°C.

8. Procédé selon la revendication 2, dans lequel ladite oxydation est effectuée à une température d'environ 110°C à environ 160°C.

9. Procédé selon la revendication 1, dans lequel ladite oxydation est effectuée sous une pression d'environ 50 psig à environ 1000 psig.

10. Procédé selon la revendication 1, dans lequel ladite oxydation est effectuée sous une pression d'environ 50 psig à environ 400 psig.

11. Procédé selon la revendication 1, dans lequel ledit pH du courant de matière première oxydable est situé dans la plage allant d'environ -4,0 à environ 1,0.

12. Procédé selon la revendication 1, dans lequel ledit pH du courant de matière première oxydable est situé dans la plage allant d'environ -1,8 à environ 1,0.

13. Procédé selon la revendication 1, dans lequel ledit pH du courant de matière première oxydable est situé dans la plage allant d'environ -1,5 à environ 1,0.
